(19) Europäisches Patentamt
European Patent Office
Office européen des brevets

(11) Publication number: 0 178 438
A1

(12) # EUROPEAN PATENT APPLICATION

(21) Application number: 85111067.6

(22) Date of filing: 02.09.85

(51) Int. Cl.⁴: C 07 D 401/12
C 07 D 403/12, C 07 D 471/04
A 61 K 31/415

(30) Priority: 19.09.84 GB 8423737
17.05.85 GB 8512517

(43) Date of publication of application:
23.04.86 Bulletin 86/17

(84) Designated Contracting States:
BE CH DE FR GB IT LI NL

(71) Applicant: BEECHAM GROUP PLC
Beecham House Great West Road
Brentford Middlesex TW8 9BD(GB)

(72) Inventor: King, Francis David
8 Heath Row Bishop's Stortford
Hertfordshire(GB)

(72) Inventor: Joiner, Karen Anne
16 The Maples Harlow
Essex CM19 4QY(GB)

(74) Representative: Jones, Pauline et al,
Beecham Pharmaceuticals Patents & Trade Marks Dept.
Great Burgh Yew Tree Bottom Road
Epsom Surrey KT18 5XQ(GB)

(54) **Novel compounds.**

(57) Compounds of the formula I, a salt, quaternized derivate or a solvate thereof:

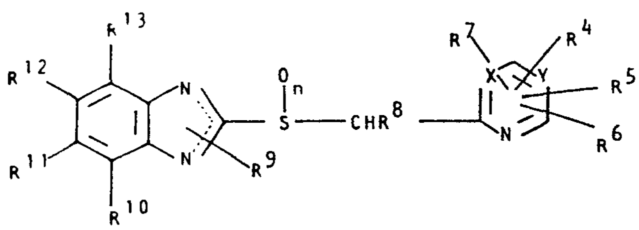

I

wherein:

n represents 0 or 1;

X and Y are both C- or one of X and Y is N and the other is CH;

$R^1$ either represents $NR^2R^3$, in which $R^2$ and $R^3$ independently represent a hydrogen atom or an alkyl, aryl, aralkyl, or acyl group, or N, $R^2$ and $R^3$ together form a saturated or unsaturated, substituted or unsubstituted ring, optionally containing one or more further hetero atoms; and

$R^4$, $R^5$, (and $R^6$ present when X and Y are both C-) each represent hydrogen or a substituent, or

$R_1$ represents $NR^2R^7$ in which $R^2$ represents a hydrogen atom or an alkyl, aryl, aralkyl, or acyl group, and $R^7$ and one of $R^4$, $R^5$, (and $R^6$ when present) on a carbon atom of the pyridine ring adjacent to that carrying $R^1$ are linked to

./...

EP 0 178 438 A1

complete a saturated or unsaturated, substituted or unsubstituted ring, optionally containing one or more further hetero atoms, and the others of $R^4$, $R^5$ (and $R^6$ when present) each represent hydrogen or a substituent;

$R^8$ represents a hydrogen atom, an alkyl group, or, when X is C and one of $R^4$, $R^5$ or $R^6$ is a substituent, $R^8$ completes a ring therewith;

$R^9$ represents a hydrogen atom, or an alkanoyl, alkoxycarbonyl, aryloxycarbonyl, aralkyloxycarbonyl, aralkanoyl, aroyl, alkylsulphonyl, arylsulphonyl, aralkylsulphonyl, or aralkyl group; and

$R^{10}$, $R^{11}$, $R^{12}$ and $R^{13}$ each independently represents hydrogen or a substituent having gastric secretion inhibition activity, a process for their preparation and their use as pharmaceuticals.

# NOVEL COMPOUNDS

This invention relates to novel compounds, to a process for their preparation and to their use as pharmaceuticals.

The present invention provides pyrimidyl and pyridyl-alkylene-sulphinylbenzimidazoles and pyrimidyl and pyridyl-alkylenethio-benzimidazoles, especially pyrimid-2-yl pyrimid-4-yl and pyrid-2-yl compounds, in which the pyrimidyl or pyridyl nucleus carries a substituted or unsubstituted amino group, and their salts, quaternized derivatives and solvates.

The compounds of the invention inhibit gastric secretion and inhibit the enzyme $(H^{+}+K^{+})$-ATPase, and may accordingly be used in the treatment of disorders caused or exacerbated by excess gastric acid secretion, for example, peptic ulcer and Zollinger-Ellison syndrome.

More especially, the invention provides a compound of the formula I a salt, quaternized derivate or a solvate thereof:

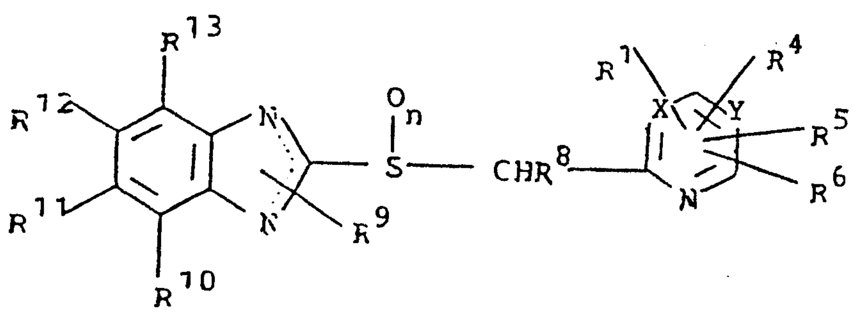

I

wherein:

n represents 0 or 1;

X and Y are both C- or one of X and Y is N and the other is CH;

$R^1$ either represents $NR^2R^3$, in which $R^2$ and $R^3$ independently represent a hydrogen atom or an alkyl, aryl, aralkyl, or acyl group, or N, $R^2$ and $R^3$ together form a saturated or unsaturated, substituted or unsubstituted ring, optionally containing one or more further hetero atoms; and

$R^4$, $R^5$, (and $R^6$ present when X and Y are both C-) each represent hydrogen or a substituent, or

$R_1$ represents $NR^2R^7$ in which $R^2$ represents a hydrogen atom or an alkyl, aryl, aralkyl, or acyl group, and $R^7$ and one of $R^4$, $R^5$, (and $R^6$ when present) on a carbon atom of the pyridine ring adjacent to that carrying $R^1$ are linked to complete a saturated or unsaturated, substituted or unsubstituted ring, optionally containing one or more further hetero atoms, and the others of $R^4$, $R^5$ (and $R^6$ when present) each represent hydrogen or a substituent;

$R^8$ represents a hydrogen atom, an alkyl group, or, when X is $C^-$ and one of $R^4$, $R^5$ or $R^6$ is a substituent, $R^8$ completes a ring therewith;

$R^9$ represents a hydrogen atom, or an alkanoyl, alkoxycarbonyl, aryloxycarbonyl, aralkyloxycarbonyl, aralkanoyl, aroyl, alkylsulphonyl, arylsulphonyl, aralkylsulphonyl, or aralkyl group; and

$R^{10}$, $R^{11}$, $R^{12}$ and $R^{13}$ each independently represents hydrogen or a substituent.

When $R^4$, $R^5$, and $R^6$ represent a substituent, they advantageously independently represent a halogen atom, or an alkyl, alkoxy, alkylthio, alkoxyalkoxy, acyloxy, cyano, nitro, carboxy, esterified carboxy, or acyl group, or $NR^2R^3$, where $R^2$ and $R^3$ have the meanings given above. When one of $R^4$, $R^5$ and $R^6$ is linked to an $R^7$ group of $R^1$ to form a ring as specified above, and one or both of the others represents a substituent, the or each independently advantageously represents a halogen atom, or an alkyl, alkoxy, alkylthio, alkoxyalkoxy, or acyloxy group, or $NR^2R^3$, where $R^2$ and $R^3$ have the meanings given above. Alternatively, when one of $R^4$,$R^5$ and $R^6$ is linked to an $R^7$ group of $R^1$ to form a ring as specified above, and the remaining two of $R^4$, $R^5$ and $R^6$ are adjacent to each other, one of the remaining two may represent $NR^2R^7$, where $R^2$ and $R^7$ have the meanings given above, $R^7$ being linked to the other of the remaining two, to form a further ring as specified above.

The preferred location of $R^1$ is in the 4-position on the pyr(im)idine ring.

Preferred examples of $R^2$ and $R^3$ are alkyl groups, especially preferred being the methyl group; particularly preferred are compounds in which both $R^2$ and $R^3$ represent methyl groups. Other preferred compounds are those in which $R^2$ and $R^3$ both represent ethyl groups, or $R^2$ represents hydrogen and $R^3$ represents the benzyl group, or $R^2$ represents the methyl and $R^3$ the phenyl group. When $NR^2R^3$ together form a ring, it is advantageously a pyrrolidine or morpholine ring, which may be substituted or unsubstituted, for example, a pyrrolidone ring, e.g., 2-pyrrolidone.

Preferred examples of $R^4$, $R^5$ and $R^6$ are hydrogen and alkyl, especially methyl. Preferred locations of the alkyl group or groups when $R^1$ is in the 4-position, are the 3- and 5-positions.

When $R^1$ represents $NR^2R^7$, the ring formed is advantageously a 5- or 6-membered ring, which may be saturated or unsaturated, and may contain a further hetero atom or atoms, especially a further nitrogen atom. Among such substituents are those in which $R_1$ is at the 4-position and which result, for example, in radicals of the formula

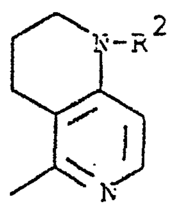
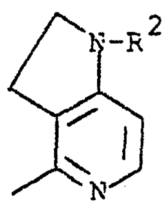
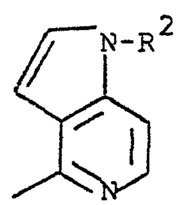
or
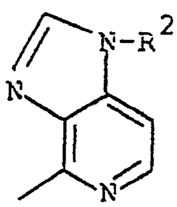

in which $R^2$ preferably represents hydrogen or a methyl group.

Preferred examples or $R^8$ are the hydrogen atom and the methyl group. When $R^8$ and one of $R^4$, $R^5$, and $R^6$ are linked to complete a ring, they advantageously together represent a alkylene group with from 2 to 9 carbon atoms and preferably form a 4 to 11 membered ring.

Preferably $R^9$ represents the hydrogen atom.

When $R^9$ represents an aralkylcarbonyl or aralkyl group, the alkylene moiety is advantageously methene.

Preferred examples of $R^{10}$, $R^{11}$, $R^{12}$ or $R^{13}$ are hydrogen or halogen atoms and substituted or unsubstituted amino, alkyl, alkoxy, alkylthio, alkoxyalkoxy, acyloxy, alkoxycarbonyl, alkanoyl, aryl or aralkyl groups or the carboxy, esterified carboxy, cyano or nitro groups, especially preferred being hydrogen, chlorine, bromine and fluorine atoms, and methyl, trifluoromethyl, methoxy, ethoxycarbonyl and acetyl groups. The substituents are preferably in the 5-position of the benzimidazole ring, and if there are two substituents these are preferably in the 5,6-positions, e.g., the 5, 6-dichloro compound. Preferably, at least two of $R^{10}$, $R^{11}$, $R^{12}$ and $R^{13}$ represent hydrogen; most preferably all four thereof represent hydrogen.

A preferred group of compounds of the formula I comprises those of the formula II

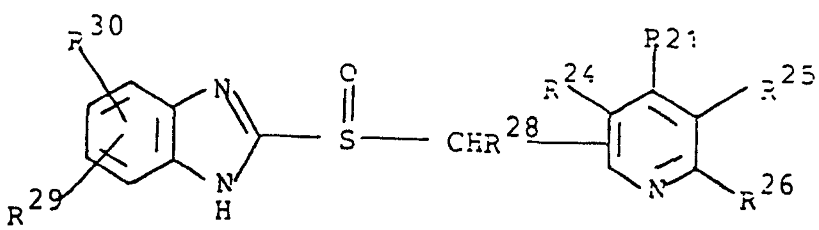

II

wherein $R^{21}$ represents dialkylamino, especially dimethylamino piperidino, pyrrolidino or morpholino; $R^{24}$, $R^{25}$ and $R^{26}$ each independently represents hydrogen or alkyl, especially methyl; $R^{28}$ represents hydrogen or methyl, and $R^{29}$ and $R^{30}$ each independently represents hydrogen, chlorine, bromine, fluorine, or an amino, methyl, trifluoromethyl, methoxy, ethoxycarbonyl or acetyl group.

A further group of compounds within formula I is of formula III:

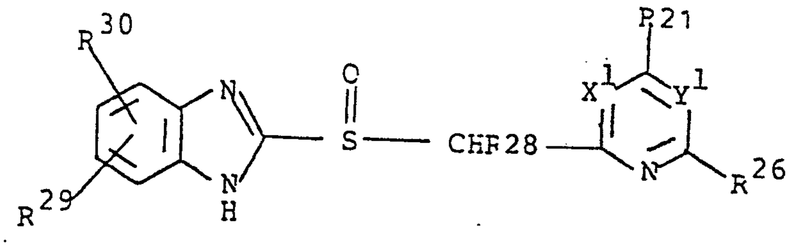 . (III)

wherein $X^1$ is N and $Y^1$ is $CR^{25}$ where $R^{25}$ is as defined in formula II or $X^1$ is $CR^{24}$ where $R^{24}$ is as defined in formula II and $Y^1$ is N.

Especially preferred compounds are those in which n = 1, $R^1$ represents dimethylamino, piperidino or morpholino, two of $R^4$, $R^5$ and $R^6$ represent methyl, the other representing hydrogen; $R^8$ represents hydrogen, and $R^9$, $R^{10}$, $R^{11}$, $R^{12}$ and $R^{13}$ each represent hydrogen. Among such compounds that in which n = 1, $R^1$ is in the 4-position on the pyridine ring and the two methyl groups among $R^4$, $R^5$ and $R^6$ are in the 3- and 5-positions thereon is preferred.

Among alkyl and alkoxy substituents, alkyl and alkoxy groups having up to six carbon atoms are preferred, groups containing up to four carbon atoms being especially preferred. The groups may have straight or branched chains or may be cyclic; the methyl, ethyl, n-propyl, and <u>tert</u>-butyl groups and $C_{3-6}$ cycloalkyl groups and the corresponding alkoxy groups being particularly preferred. Among acyl groups, carboxylic acyl groups containing from 1 to 7, especially 1 to 5, carbon atoms are preferred.

In formula I, the symbol

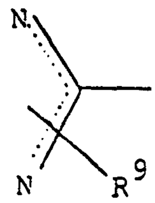

means that $R^9$ may be attached to either of the nitrogen atoms of the imidazole ring and that, when $R^9$ represents hydrogen, tautomerism exists; both tautomers are included within the scope of this invention. When $R^9$ does not represent hydrogen, the two compounds may differ because of the asymmetry in the benzene ring substituents, and both compounds are included within the scope of this invention.

The compounds of the invention may have chiral centres, and be capable of existing in a number of stereoisomeric forms. The invention extends to each of these stereoisomeric forms, including enantiomers, and to mixtures thereof, including racemates.

Examples of chiral centres include the sulphur atom when n = 1, and the carbon atom of $CHR^8$ when $R^8$ is other than H.

Of compounds of the invention, especially those of formula I, their salts, quaternized derivatives and solvates, those that are pharmaceutically acceptable are preferred, and the present invention accordingly also provides such compounds.

Pharmaceutically acceptable salts of the compounds of the formula I include pharmaceutically acceptable salts of inorganic acids, for example, the sulphate, nitrate, phosphate, borate, hydrochloride and hydrobromide, and pharmaceutically acceptable organic addition salts, for

example, acetate, fumarate, tartrate, citrate, lactate, salicylate, maleate, succinate, benzoate, ascorbate, methanesulphonate, mandelate, α-ketoglutarate, α-glycerophosphate, and glucose-1-phosphate. The preferred acid addition salt is the hydrochloride salt.

Examples of quaternized derivatives include compounds of the formula I quaternized by $R^{14}Q$, where $R^{14}$ represents alkyl, cycloalkyl or phenyl-alkyl and Q is halide, for example, chloride, bromide or iodide. The alkyl radicals in such groups advantageously contain up to 4 carbon atoms, the cycloalkyl radcals advantageously contain from 3 to 6 carbon atoms.

A compound of the formula I or its acid addition salt may form salts with alkali and alkaline earth metals, suitably sodium and potassium, and ammonium and substituted ammonium salts, and such compounds are included within the scope of the invention.

Crystalline compounds and salts are preferred.

Pharmaceutically acceptable compounds of formula I, pharmaceutically acceptable salts and quarternized derivates thereof, and pharmaceutically acceptable solvates of any of the foregoing may be used in the treatment of disorders caused or exacerbated by excess gastric acid secretion such as peptic ulcer and Zollinger-Ellison syndrome.

The present invention also provides a process for the preparation of a compound according to the invention by reaction of a 2-substituted benzimidazole with a substituted pyridine or pyrimidine wherein the substituents on the two compounds react to provide a linkage of the formula IV

$$-S(O)_nCHR^8- \qquad (IV)$$

with the sulphur atom on the 2-position of the benzimidazole nucleus, where n and $R^8$ have the meanings given above, and, if desired or required, providing or removing other substituents in or from the resulting compound and, if desired or required, removing protective groups from other substituents on the resulting compound and, if desired or required, oxidizing or reducing the resulting compound, and, if desired or required, converting a free base into an acid addition salt, or quaternizing or solvating the resulting compound.

Examples of the reaction are reactions in which

(a) the benzimidazole is a 2-thiol and the pyridine or pyrimidine carries a $-CHR^8R^{31}$group, where $R^{31}$ is a leaving group, especially one displaceable by a nucleophile, and $R^8$ has the meaning given above;

(b) the benzimidazole carries at the 2-position a group of the formula $-S(O)H_2R^8$ or $-S(O)CHR^8R^{32}$ where R has the meaning given above and $R^{32}$ is a cationic substituent and the pyridine or pyrimidine carries a leaving group, especially one displaceable by a nucleophile; or

(c) the benzimidazole is 2-substituted by a leaving group, especially one displaceable by a nucleophile, and the pyridine or pyrimidine carries a group of the formula $CH_2R^8$ or $-CHR^8SR^{32}$, where $R^8$ and $R^{32}$ have the meaning given above.

The leaving group $R^{31}$ may be, for example, a halogen atom, for example chlorine, bromine or iodine, or a labile acyloxy group, for example $OSO_2CH_3$ or $OSO_2p\text{-}C_6H_4CH_3$, as may the leaving group on the pyridine or pyrimidine in Reaction (b) and that on the benzimidazole in Reaction (c); the latter may alternatively be a carboxylic acyloxy group, for example an alkanoyloxy group having from 1-4 carbon atoms.

The cationic substituent $R^{32}$ in Reaction (b) or (c) is advantageously an alkali metal, for example, potassium, sodium or lithium; in Reaction (c) it may however be, for example, a tetraalkylammonium group, eg., the tetraethylammonium group.

Reaction (a),(b), or (c) is generally affected at a moderate temperature, for example, a moderately elevated temperature, e.g., solvent reflux temperature, for example 50 to 150°C, especially 75 to 100°C, in an inert solvent, preferably in the presence of an acid acceptor. The acceptor is suitably an inorganic acid acceptor, for example a strong base, e.g., sodium hydride, potassium t-butoxide, butyl lithium or lithium diisopropylamide; a moderately strong base for example sodium hydroxide; or a moderate base for example calcium carbonate, sodium carbonate or potassium carbonate. In some cases the moderate base acid acceptor may suitably be an organic base, for example,

a tertiary amine, e.g., triethylamine, trimethylamine, pyridine or picoline. The appropriate acceptor depends on the particular reaction and, it will be appreciated, may provide the cationic substituent $R^{32}$ mentioned above. For example, for Reaction (b) a strong base is appropriate. For reaction (a) a moderately strong base or moderate base is appropriate, while for Reaction (c) a moderate base is adequate.

The inert solvent may be any solvent inert to both reactants and appropriate to the leaving group, the acid acceptor if present and desired reaction temperature. Suitable solvents include lower alkanols, for example, ethanol, dioxan, dimethyl formamide (DMF), toluene, diethyl ether, or methylene chloride.

For Reaction (a) in particular suitable solvents include polar solvents, for example, DMF or ethanol. Where reaction is effected in the presence of a base which is insoluble in the polar solvent, water, may be added to the solvent. Reaction is generally effected at moderately elevated temperatures as mentioned hereinbefore, for example, reaction mixture reflux temperature or at about 100°C, if lower.

It will be appreciated that when the compound of the invention to be produced is to contain an unsubstituted amino group, such a group will generally be protected, during at least the main reaction of the invention, by a conventional N-protecting group, for example, an acyl group, e.g., acetyl or phthaloyl. Protection may be effected by reaction with an acylating agent, for example the relevant acyl chloride or anhydride. The protecting group may be removed, for example, by base hydrolysis of an acetyl protecting group or treatment of a phthalimide protecting group with hydrazine hydrate in a lower alkanol, such as ethanol.

Advantageously, the process results in a compound of the formula I, and preferably in a compound of the formula II or of formula III.

If the reaction produces a compound of the formula I wherein n is 0 and a compound of the formula I is desired wherein n = 1, it is necessary to oxidize the compound resulting from the reaction.

Such oxidation may be carried out, for example, at below ambient temperatures in non-aqueous solvent, for example, a chlorinated hydrocarbon using, for example, an organic peracid, e.g., 3-chloroperbenzoic acid, or in water in the presence of a soluble strong inorganic oxidant, for example hydrogen peroxide.

It will be realized that this process may also N-oxidize any tertiary amino moiety and suitable precautions should be taken if it is desired to avoid this.

Conversely, reduction is required if the reaction produces a compound of the formula I in which m is 1 and a compound in which n is 0 is required.

It will be apparent that compounds of the formula I containing a substituent which is convertible to another substituent wherein the resulting compound is of the formula I are useful novel intermediates. Numerous such conversions are possible not only for the end compounds of formula I, but also for their intermediates as follows:

(a) an acyl substituent is convertible to a hydrogen atom, or a diacyl substituent is convertible to two hydrogen atoms, by deacylation;

(b) a hydrogen atom is convertible to an acyl substituent, or two hydrogen atoms are convertible to a diacyl substituent, by acylation;

(c) an alkyl substituent is convetible to a hydrogen atom, or a dialkyl substituent is convertible to two hydrogen atoms, by dealkylation;

(d) a hydrogen atom is convertible to an alkyl substituent, or two hydrogen atoms are convertible to a dialkyl substituent by alkylation.

In (a) and (b), the terms acyl and diacyl respectively include all the radicals and diradicals derivable from mono- and dibasic acids, whether carboxylic or non-carboxylic.

In (a), deacylation is desirably carried out by treatment with a base, for example, an alkali metal hydroxide.

In (b), the acylation is desirably carried out with an acylating agent, for example the corresponding acid anhydride or dianhydride or acid chloride or dichloride. Formylation is desirably carried out with formic acid.

In (c) and (d) the term alkyl includes substituted alkyl, for example, phenylalkyl, especially phenyl-lower alkyl.

In (c), dealkylation is advantageously carried out conventionally with a base, for example an alkali metal hydroxide, or, when $R_2$ or $R_3$ is benzyl, by hydrogenolysis using, for example, conventional transition-metal catalyzed hydrogenolysis.

In (d), alkylation is advantageously effected conventionally with an alkylating agent, for example, the corresponding labile ester, for example the mesylate or tosylate, or diester or the corresponding halide, for example, the bromide, or dihalide.

For $R^9$

(e) hydrogen is desirably converted to acyl by conventional acylation.

(f) an acyl group is desirably converted to hydrogen by conventional deacylation.

In (e), N-acylation is desirably carried out under conventional conditions with an acylating agent which has an acyl group capable of forming a hydrolyzable acyloxy group and a leaving group, for example halide, e.g., chloride and bromide, and hydrogen. When halide is the leaving group, the reaction is generally carried out in the presence of a base. When hydrogen is the leaving group, the reaction is generally carried out in the presence of a dehydrating agent, at a moderate temperature, such as ambient temperature.

In (f), deacylation is desirably carried out by treatment with a base, e.g., an alkali metal hydroxide.

For $R^{10}$ to $R^{13}$

(g) a hydrogen atom is convertible to a nitro substituent by nitration.

(h) a hydrogen atom is convertible to a halo substituent by halogenation;

(i) an amino substituent is convertible to a halo, cyano, or hydrogen substitute n by diazotisation and simultaneous nitrogen elimination with halogenation, cyanation, or reduction.

(j) a carboxy substituent is convertible to an alkoxycarbonyl substituent by esterification.

In (g), nitration is advantageously carried out in accordance with known procedures.

In (h) halogenation is advantageously carried out with conventional halogenating agent, under conventional reaction conditions for the halogenation of aromatic nuclei, for example, with the relevant halogen in the presence of Lewis acid catalyst, for example ferric chloride, zinc chloride or boron trifluoride, in an inert organic solvent, for example chloroform or dichloromethane, at a temperature below ambient acectic acid without a catalyst, may be used.

(i) may be effected, for example, by treating the reaction product with nitrite and a strong inorganic acid in aqueous solution at 10 to -10°C.

Subsequent simultaneous nitrogen elimination and halogenation, cyanation or reduction may be effected by treating the diazotisation product with a halide, cyanide or hydride source, under either Sandmeyer or Schiemann reaction conditions for halide and nitrile, or by treatment with hypophosphorous acid for hydride respectively.

(j) may be effected entirely conventionally.

Conversions (a) to (j) are only exemplary and are not exhaustive of the possibilities.

In general, interconversion of $R^4$, $R^5$ and $R^6$ to other $R^4$, $R^5$ and $R^6$ will not be effected. However, such interconversions as are possible, and suitable methods and conditions for effecting them, will be readily apparent to one skilled in the art.

In all the foregoing interconversions, the effect, if any, on other substituents should be borne in mind, and such reagents as are appropriate should be selected together with the adoption of such routine precautionary measures as are necessary.

It is however generally preferred that any conversions are carried out at the earliest stage possible in the synthesis.

Salts of compounds of the formula I may generally be made in an entirely conventional manner by reacting a compound of the formula I in base form with a chosen acid to form acid addition salts.

The quaternary derivatives of the compounds of the formula I may generally be prepared in conventional manner, such as by reaction of the chosen compound of the formula I with a compound $R^{14}Q$ wherein $R^{14}$ and Q are as given above. This reaction is suitably carried out in an appropriate solvent, for example, acetone, methanol or ethanol.

Salts of compounds of the formula I containing a carboxy group may generally be formed conventionally by reacting a compound of the formula I with a corresponding base, for example an alkali metal hydroxide, an alkaline earth metal hydroxide or an optionally substituted ammonium hydroxide.

The preparation of intermediates for the above preparative processes may be effected by building up the intermediates in any given process by processes analogous to those in similar processes or by conventional oxidation as described hereinbefore for a compound of the formula I, or such intermediates are known compounds or are preparable analogously to or routinely derivable from known compounds.

However, by way of example the preparation of various intermediates are as follows hereinafter:
In Reaction (a), the 2-thiol may be prepared by treating an ortho-diamino benzene with carbon disulphide or potassium ethyl xanthate (see Organic Syntheses, (1963) Collective Volume IV, 569) or by treating a benzimidazole having at the 2-position a group displaceable by a nucleophile with sodium sulphide.

In Reaction (b), the benzimidazole intermediate bearing a $-S(O)CHR^8R^{32}$ group in the 2-position may be prepared by oxidizing, in the manner described above for a compound of formula I, a corresponding compound bearing a $-SCHR^8R^{32}$ group. This compound may be prepared by conventional S-alkylation of the 2-thiol.

The invention accordingly also provides a pharmaceutical composition comprising a pharmaceutically acceptable compound of the invention, in particular a compound of formula I, a pharmaceutically acceptable salt or quaternized derivative thereof, or a pharmaceutically acceptable solvate of any of the foregoing, together with a pharmaceutically acceptable carrier.

The compositions may be formulated for administration by any route, although oral administration is preferred. The compositions may be in the form of tablets, capsules, powders, granules, lozenges, suppositories, reconstitutable powders, or liquid preparations, for example oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients, for example, binding agents, for example syrup, acacia, gelatin, sorbitol, tragacanth, or polyvinylpyrrolidone; fillers, for example lactose, suger, maize-starch, calcium phosphate, sorbitol or glycine; tabletting lubricants, for example magnesium stearate, disintegrants, for

example starch, polyvinylpyrrolidone, sodium starch glycollate or microcrystalline cellulose; or pharmaceutically acceptable wetting agents, for example, sodium lauryl sulphate.

Solid compositions for oral administration may be prepared by convention methods, for example, blending, filling, or tabletting. Repeated blending operations may be used to distribute the active agent throughout those compositions employing large quantities of fillers. The tablets may be coated according to methods well known in normal pharmaceutical practice, in particular with an enteric coating.

Liquid preparations for oral administration may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles, which may include edible oils, for example almond oil and fractionated coconut oil; oily esters, for example esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, may be either suspended or dissolved in the vehicle. In

preparing solutions the compound may be dissolved in water for injection and filter sterilized before filling into a suitable vial or ampoule and sealing. Advantageously, adjuvants, for example, a local anaesthetic, a preservative and buffering agents may be dissolved in the vehicle. To enchance the stability, the composition may be frozen after filing into the vial and the water removed under vacuum. Parenteral suspensions are prepared in substantially the same manner, except that the compound is suspended in the vehicle instead of being dissolved, and sterilization cannot be accomplished by filtration. The compound may be sterilized by exposure to ethylene oxide before suspending in the sterile vehicle. Advantageously, a surfactant or wetting agent is included in the composition to facilitate uniform distribution of the compound.

When appropriate the compositions of this invention may be presented as an aerosol for oral administration, as a microfine powder for insufflation, or as a suppository for rectal or vaginal administration. Suitable unit dosage forms include tablets, capsules and powders in sachets or vials, and preferred forms include shaped oral unit doses, for example tablets and capsules.

The compositions may contain from 0.1% to 99% by weight, preferably from 10-60% by weight, of the active material, depending on the method of administration.

The invention also provides a method of treatment or prophylaxis of disorders, for example peptic ulcers, in animals,especially mammals, including humans, caused or exacerbated by excess gastric secretation, which comprises the administration of an effective amount of

a compound of the present invention, in particular a compound of formula I, or a pharmaceutically acceptable salt or quaternized derivative thereof, or a pharmaceutically acceptable solvate of any of the foregoing, advantageously in admixture with a pharmaceutically acceptable carrier, to the sufferer.

The amount effective to treat the disorders hereinbefore described depends on the relative efficacies of the compounds of the invention, the nature and severity of the disorder being treated and the weight of the animal. However, a unit does will normally contain 1 to 2000, for example 5 to 1000, mg of the compound of the invention. Unit doses will normally be administered at least once a day, for example, 1,2,3,4,5 or 6 times a day such that the total daily dose is normally in the range 0.1 to 30 mg/kg per day, for example 7 to 2000 mg/day for a 70kg human adult.

No adverse toxicological effects are indicated at the aforementioned dosage ranges.

The compositions may be accompanied by written or printed directions for use in the medical treatment concerned.

The invention also provides a compound of the present invention, in particular a pharmaceutically acceptable compound of the formula I or a pharmaceutically acceptable salt or quaternized derivative thereof, or a pharmaceutically acceptable solvate of any of the foregoing for use as an active therapeutic substance; in particular for use in the treatment or prophylaxis of disorders those caused or exacerbated by excess gastric secretion.

The following Descriptions illustrate the preparation of intermediates. The following Examples illustrate the invention.

Description 1

4-Dimethylamino-2,3,5-trimethylpyridine

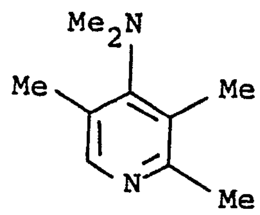

(D1)

A solution of 2,3,5-trimethyl-4-nitropyridine N-oxide (1.5 g) and dimethylamine hydrochloride (10 g) in N-methylpyrrolidone (20 ml) was heated to 190°C for 1 h. On cooling, the reaction mixture was poured into ice and made alkaline with potassium carbonate. The product was extracted into ether (3 x 70 ml), dried and concentrated. The residue was purified by column chromatography (silica, ether) to give the title compound (0.6 g, 40%).

$^1$H-NMR ($CDCl_3$)

δ = 7.97 (s, 1H)
2.77 (s, 6H)
2.40 (s, 3H)
2.15 (brs. 6H)

Description 2

4-Dimethylamino-2,3,5-trimethylpyridine-N-oxide

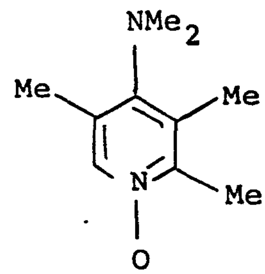

(D2)

To a stirred solution of 4-dimethylamino-2,3,5-trimethylpyridine (0.9 g) in $CHCl_3$ (50 ml) was added 1.1 g m-chloroperoxybenzoic acid. After heating under reflux for 1 hr, the reaction mixture was treated with aqueous $NaHCO_3$ solution, the chloroform layer separated and dried ($Na_2SO_4$). Concentration afforded the title compound (ca, 1.0 g, 100%) as an oil.

$^1H$-NMR ($CDCl_3$)

$\delta$ = 7.87 (s, 1H)
2.76 (s, 6H)
2.43 (s, 3H)
2.16 (brs. 6H)

Description 3

4-Dimethylamino-3,5-dimethyl-2-hydroxymethyl-pyridine

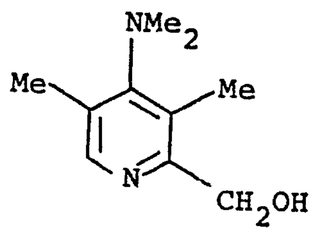

(D3)

A solution of 4-dimethylamino-2,3,5-trimethylpyridine-N-oxide (2.1 g) in acetic anhydride (50 ml) was heated on steam bath for 2 hr. After concentration *in vacuo*, methanol (50 ml), followed by aqueous NaOH (2.5N, 20 ml), was added and the mixture heated under reflux for 1 h. On concentration, the aqueous residue was saturated with potassium carbonate and extracted with ether (3 x 70 ml). Drying ($Na_2SO_4$) and evaporation afforded the title compound (1.9 g, 90%) as an oil.

$^1H$-NMR ($CDCl_3/D_2O$)

$\delta$ = 8.00 (s, 1H)
4.51 (s, 2H)
2.80 (s, 6H)
2.22 (s, 3H)
2.06 (s, 3H)

Description 4

4-Dimethylamino-2-chloromethyl-3,5-dimethylpyridine hydrochloride

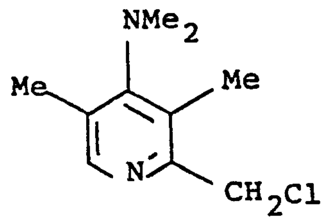

(D4)

Thionyl chloride (5 ml) was added to a solution of 4-dimethylamino-3,5-dimethyl-2-hydroxymethylpyridine (1.9 g) in chloroform (50 ml) at room temperature. After heating under reflux for 2 h, the solvent was evaporated *in vacuo* and the residue triturated under xylene (20 ml) and re-evaporated. Trituration of the residue with ether gave the 4-dimethylamino-2-chloromethyl-3,5-dimethylpyridine hydrochloride (2.2 g, 88%).

Description 5

5,6-Dimethyl-4-(1-piperidinyl)pyrimidine-N-oxide (D5)

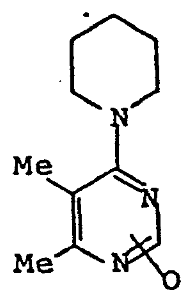

(D5)

Meta-chloroperoxybenzoic acid (mCPBA) (1.45g) was added to a solution of 4-chloro-5,6-dimethylpyrimidine (1.0g) in $CHCl_3$ (20ml) and the solution heated under reflux for 3h. A further quantity of mCPBA (0.3g) was added and heating was continued for a further 1h. The reaction mixture was cooled and treated with a solution of piperidine (3.6g) in $CHCl_3$ (20ml). After 24h, the reaction mixture was evaporated to dryness and the residue partitioned between 10% sodium carbonate solution and $CHCl_3$ (100ml). The organic layer was dried ($Na_2SO_4$), concentrated and the residue purified by column chromatography on silica, eluting with $CHCl_3$ to give D5 (0.6g).

Description 6

6-Hydroxymethyl-5-methyl-4-(1-piperidinyl)pyrimidine (D6)

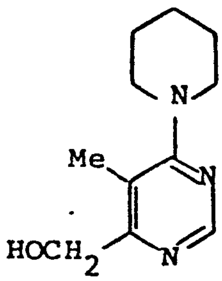

(D6)

A solution of D1 (3.5g) in toluene (30ml) and acetic anhydride (20ml) was heated under reflux for 2h. The reaction mixture was then evaporated to dryness and the residue purified by column chromatography on silica, eluting with $CHCl_3$ to give the 6-acetoxymethyl derivative (1.0g). The 6-acetoxymethyl derivative (0.93g) was heated to reflux in EtOH (10ml) containing 10% aqueous sodium hydroxide solution (2.7ml) for 2h. The mixture was evaporated to dryness, and the residue partitioned between water (5ml) and $CHCl_3$ (100ml). The organic layer was dried ($Na_2SO_4$), evaporated to dryness and the residue purified by column chromatography on silica, eluting with $CHCl_3$, to give D6 (0.52g).

Description 7

6-Chloromethyl-5-methyl-4-(1-piperidinyl)pyrimidine hydrochloride (D7)

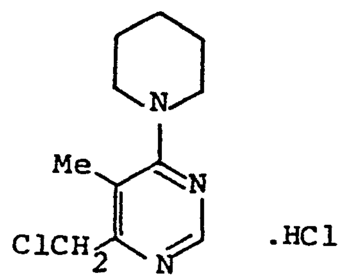

(D7)

A $CHCl_3$ (15ml) solution of D2 (0.48g) was treated with thionyl chloride (0.7g) and then heated under reflux for 1h. The reaction mixture was evaporated *in vacuo* and the residue azeotroped with toluene (2 x 50ml) to give D7 (0.56g).

EXAMPLE 1

2-[ (4-Dimethylamino-3, 5-dimethylpyrid-2-yl) methylthio]-benzimidazole

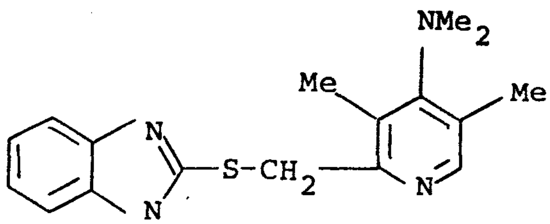

(E1 )

To 4-dimethylamino-2-chloromethyl-3, 5-dimethylpyridine hydrochloride (0.47 g) and 2-mercaptobenzimidazole (0.3 g) in ethanol (50 ml) was added 10% sodium hydroxide solution (1.6 ml). After heating under reflux for 3 h, the cooled reaction mixture was evaporated and the residue partitioned between chloroform (100 ml) and water (20 ml). The chloroform layer was separated, dried ($Na_2SO_4$) and concentrated. The residue was purified by column chromatography (silica, chloroform) to give the title compound (0.35 g) as a white solid (56%) (mp 149-50$^oC$).

$^1N$-NMR ($CDCl_3$)

| | | |
|---|---|---|
| δ= | 7.98 | (s, 1H) |
| | 7.5-6.8 | (m, 4H) |
| | 4.33 | (s, 2H) |
| | 4.73 | (s, 6H) |
| | 2.21 | (s, 3H) |
| | 2.13 | (s, 3H) |

EXAMPLE 2

2-[ (4-Dimethylamino-3, 5-dimethylpyrid-2-1) methylsulphinyl] benzimidazole

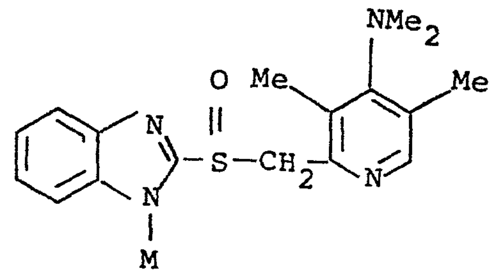

(E2)

A solution of 2-[ (4-dimethylamino-3, 5-dimethylpyrid-2-yl)methylthio] benzimidazole (0.31 g) and m-chloroperoxy-benzoic acid (0.19 g) in $CHCl_3$ (100 ml) was stirred at 0°C for 10 min. After treatment with aqueous sodium bicarbonate solution, the separated chloroform layer was fired ($MgSO_4$) and concentrated *in vacuo*. Trituration with acetonitrile afforded the title compound (0.18 g) as a grey solid (55%) (mp 142-4°C dec).

$^1$H-NMR ($CD_2Cl_2$)

| | | |
|---|---|---|
| δ= | 8.14 | (s, 1H) |
| | 7.70-7.45 | (m, 2H) |
| | 7.40-7.15 | (m, 2H) |
| | 4.73 | (s, 2H) |
| | 2.76 | (s, 6H) |
| | 2.24 | (s, 3H) |
| | 2.15 | (s, 3H) |
| | 2.15 | (s, 3H) |

Example 3a

5-Methyl-2-[(4-dimethylamino-3,5-dimethylpyrid-2-yl)-methylthio]benzimidazole (E3a)

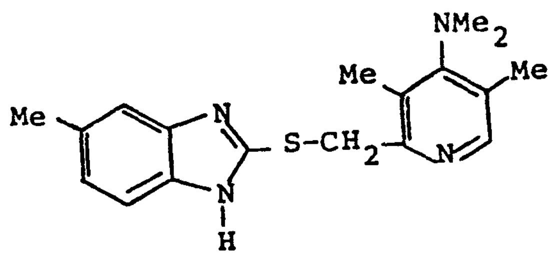

(E.3a)

$^1$H-NMR ($CDCl_3$)

| | | |
|---|---|---|
| δ | 8.06 | (s, 1H) |
| | 7.4-7.1 | (m, 2H) |
| | 6.86 | (d, 1H) |
| | 4.25 | (s, 1H) |
| | 2.80 | (s, 6H) |
| | 2.40 | (s, 3H) |
| | 2.25 | (s, 3H) |
| | 2.21 | (s, 3H) |

Example 3b

5-Methyl-2-[(4-dimethylamino-3,5-dimethylpyrid-2-yl)-methylsulphinyl]benzimidazole (E3b)

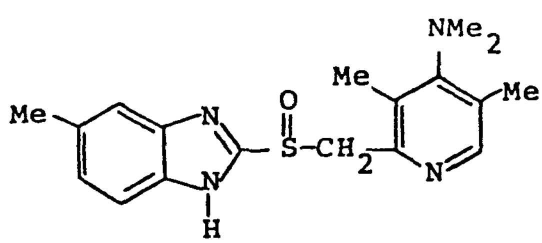

(E.3b)

$^1H$-NMR ($CD_2Cl_2$)

δ 8.14 (s, 1H)
7.49 (d, 1H)
7.35 (brs, 1H)
7.12 (brd, 1H)
4.70 (s, 1H)
2.76 (s, 6H)
2.46 (s, 3H)
2.23 (s, 3H)
2.13 (s, 3H)

Example 4a

5-Methoxy-2-[(4-dimethylamino-3,5-dimethylpyrid-2-yl)-methylthio]benzimidazole (E4a)

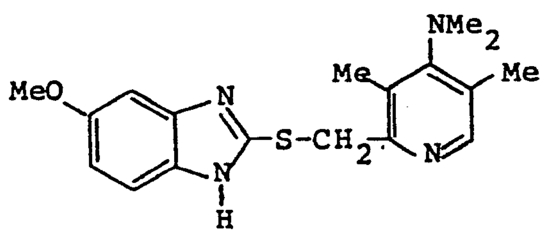

(E.4a)

$^1H$-NMR ($CDCl_3$)

δ 8.16 (s, 1H)
7.40 (d, 1H)
7.04 (d, 1H)
6.80 (dd, 1H)
4.34 (s, 2H)
3.85 (s, 3H)
2.87 (s, 6H)
2.30 (s, 3H)
2.26 (s, 3H)

Example 4b

5-Methoxy-2-[(4-dimethylamino-3,5-dimethylpyrid-2-yl)methylsulphinyl]benzimidazole (E4b)

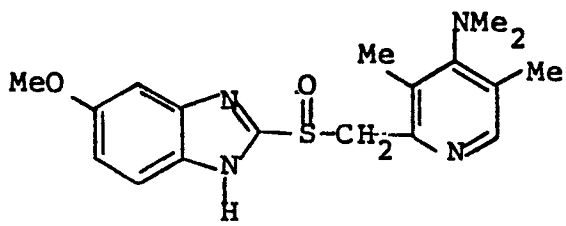

(E.4b)

$^1$H-NMR ($CD_2Cl_2$)

| | | |
|---|---|---|
| δ | 8.12 | (s, 1H) |
| | 7.49 | (d, 1H) |
| | 6.80-7.10 | (m, 2H) |
| | 4.71 | (s, 2H) |
| | 3.82 | (s, 3H) |
| | 2.75 | (s, 6H) |
| | 2.21 | (s, 3H) |
| | 2.12 | (s, 3H) |

Example 5a

5-Trifluoromethyl-2-[(4-dimethylamino-3,5-dimethylpyrid-2-yl)methylthio]benzimidazole (E5a)

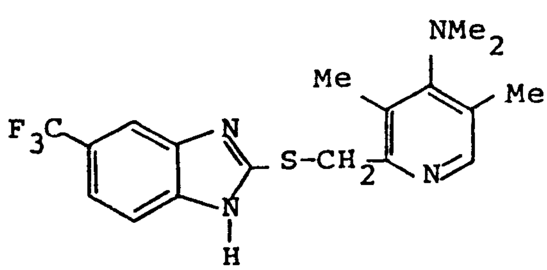

(E.5a)

Example 5b

5-Trifluoromethyl-2-[(4-dimethylamino-3,5-dimethyl-pyrid-2-yl)methylsulphinyl]benzimidazole (E5b)

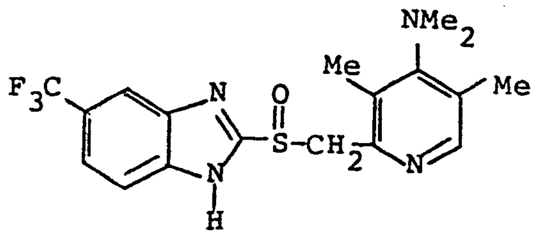

(E.5b)

Example 6a

2-[(3,5-dimethyl-4-(piperid-1-yl)pyrid-2-yl)methylthio] benzimidazole (E6a)

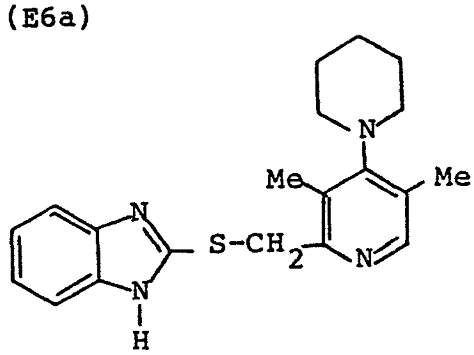

(E.6a)

$^{1}H$-NMR ($CDCl_3$)

δ 8.07 (s, 1H)
7.60-7.30 (m, 2H)
7.25-6.90 (m, 2H)
4.29 (s, 2H)
3.10-2.80 (m, 4H)
2.28 (s, 3H)
2.23 (s, 3H)
1.80-1.40 (m, 6H)

Example 6b

2-[(3,5-dimethyl-4-(piperid-1-yl)pyrid-2-yl)methyl-sulphinyl]benzimidazole (E6b)

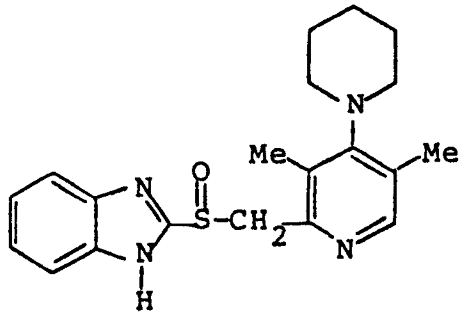

(E.6b)

$^1H$-NMR ($CDCl_3$)

| | | |
|---|---|---|
| δ | 8.14 | (s, 1H) |
| | 7.75-7.50 | (m, 2H) |
| | 7.40-7.10 | (m, 2H) |
| | 4.75 | (s, 2H) |
| | 3.00-2.70 | (m, 4H) |
| | 2.25 | (s, 3H) |
| | 2.15 | (s, 3H) |
| | 1.75-1.40 | (m, 6H) |

Example 7a

2-[(3,5-dimethyl-4-(pyrrolidin-1-yl)pyrid-2-yl)methyl-thio]benzimidazole (E7a)

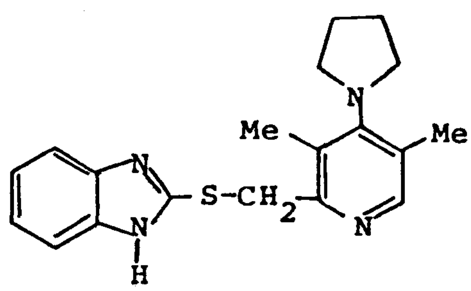

(E.7a)

NMR ($CDCl_3$)

δ 8.06 (s, 1H)
7.50-7.30 (m, 2H)
7.20-6.90 (m, 2H)
4.28 (s, 2H)
3.30-3.00 (m, 4H)
2.22 (s, 3H)
2.18 (s, 3H)
2.15-1.75 (m, 4H)

Example 8a

2-[(3,5-dimethyl-4-morpholino-pyrid-2-yl)methylthio]-benzimidazole (E8a)

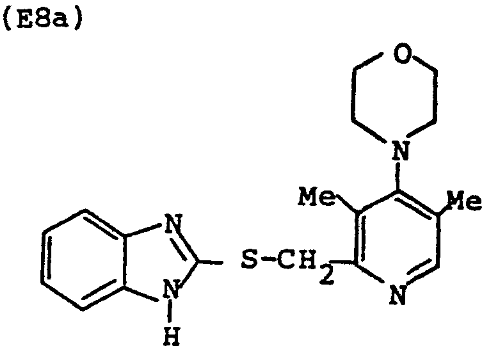

$^1H$-NMR ($CDCl_3$) (E.8a)

δ 8.06 (s, 1H)
7.55-7.25 (m, 2H)
7.20-6.90 (m, 2H)
4.31 (s, 2H)
3.85-3.65 (m, 4H)
3.20-2.90 (m, 4H)
2.31 (s, 3H)
2.27 (s, 3H)

Example 8b

2-[(3,5-dimethyl-4-morpholino-pyrid-2-yl)methyl-sulphinyl]benzimidazole (E8b)

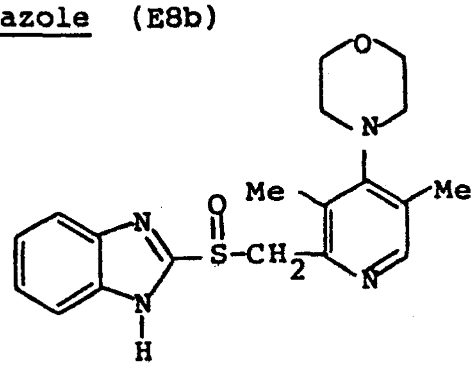

(E.8b)

$^1$H-NMR ($CDCl_3$)

| | | |
|---|---|---|
| δ | 8.18 | (s, 1H) |
| | 7.75-7.50 | (m, 2H) |
| | 7.42-7.20 | (m, 2H) |
| | 4.77 | (s, 2H) |
| | 3.82-3.57 | (m, 4H) |
| | 3.07-2.82 | (m, 4H) |
| | 2.27 | (s, 3H) |
| | 2.17 | (s, 3H) |

Example 9a

2-[(1H-imidazo(4,5-c)pyrid-6-yl)methylthio]benzimidazole (E9a)

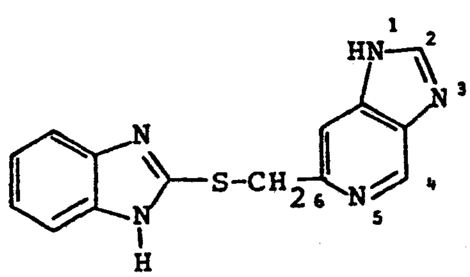

(E.9a)

Example 9b

2-[(1H-imidazo(4,5-c)pyrid-6-yl)methylsulphinyl]benzimidazole (E9b)

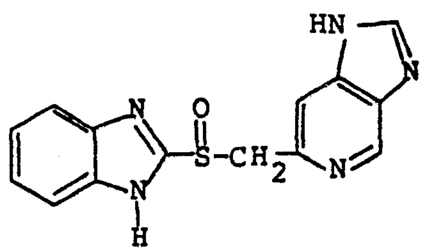

(E.9b)

Example 10a

5-Amino-6-bromo-2-[(4-dimethylamino-3,5-dimethylpyrid-2-yl)methylthio]benzimidazole (E10a)

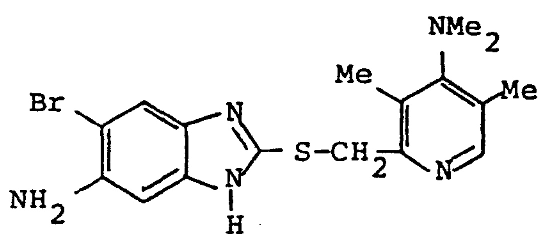

(E.10a)

NMR ($CDCl_3$)

δ 8.14 (s, 1H)
7.64 (s, 1H)
6.94 (s, 1H)
4.32 (s, 2H)
2.90 (s, 6H)
2.30 (s, 3H)
2.27 (s, 3H)

Found $M^+$ 405.0632 $C_{17}H_2N_5SBr$ requires 405.0623

Example 10b

5-Amino-6-bromo-2-[(4-dimethylamino-3,5-dimethylpyrid-2-yl)methylsulphinyl]benzimidazole (E10b)

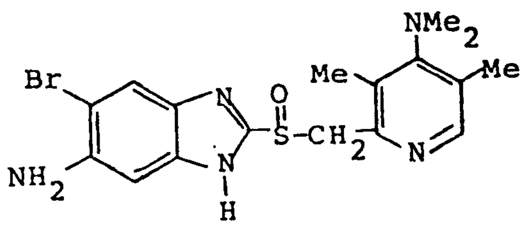

(E.10b)

NMR ($CD_2Cl_2$/$d^6$DMSO)

δ 8.10 (s, 1H)
7.75 (s, 1H)
7.02 (s, 1H)
4.61 (s, 2H)
2.84 (s, 6H)
2.24 (s, 3H)
2.18 (s, 3H)

Example 11a

5-Chloro-2-[(4-dimethylamino-3,5-dimethylpyrid-2-yl)-methylthio]benzimidazole (E11a)

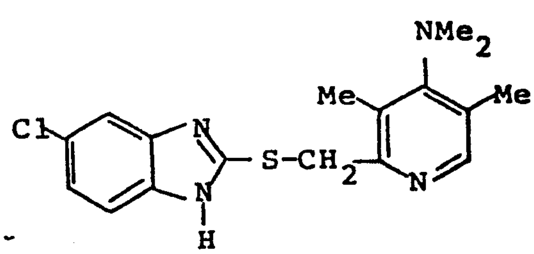

(E.11a)

NMR ($CDCl_3$)

δ 8.05 (s, 1H)
7.50-6.90 (m, 3H)
4.23 (s, 2H)
2.80 (s, 6H)
2.23 (s, 3H)
2.20 (s, 3H)

Example 11b

5-Chloro-2-[(4-dimethylamino-3,5-dimethylpyrid-2-yl)-methylsulphinyl]benzimidazole (E11b)

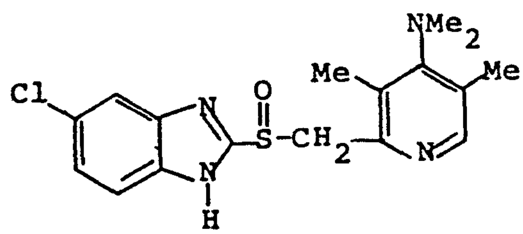

(E.11b)

NMR ($d^6$DMSO)

8.08 (s, 1H)
7.75 (s, 1H)
7.67 (d, 1H)
7.35 (dd, 1H)
4.73 (s, 1H)
2.80 (s, 6H)
2.24 (s, 3H)
2.18 (s, 3H)

Example 12a

5-Amino-6-trifluoromethyl-2-[(4-dimethylamino-3,5-dimethylpyrid-2-yl)methylthio]benzimidazole (E12a)

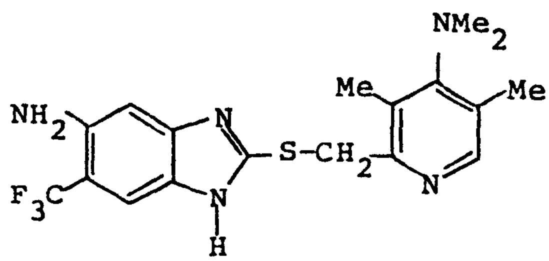

(E.12a)

NMR ($CDCl_3$)

δ 8.08 (s, 1H)
7.58 (s, 1H)
6.78 (s, 1H)
4.28 (s, 2H)
2.82 (s, 6H)
2.25 (brs, 6H)

Example 12b

5-Amino-6-trifluoromethyl-2-[(4-dimethylamino-3,5-dimethylpyrid-2-yl) methylsulphinyl]benzimidazole (E12b)

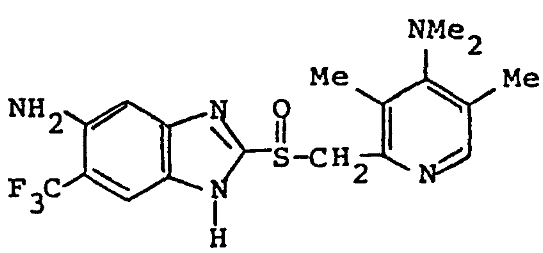

(E.12b)

NMR ($d^6$-DMSO)

δ 8.07 (s, 1H)
7.74 (s, 1H)
7.02 (s, 1H)
5.35 (brs, 2H)
4.68 (s, 2H)
2.78 (s, 6H)
2.21 (s, 3H)
2.16 (s, 3H)

Example 13a

5-Chloro-2-[(4-dimethylamino-5-methylpyrid-2-yl)methylthio]benzimidazole (E13a)

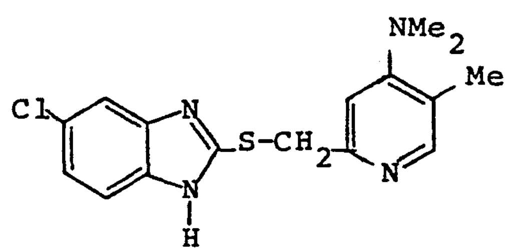

(E.13a)

NMR ($CDCl_3$)

δ 8.18 (s, 1H)
7.53 (d, 1H)
7.33 (d, 1H)
7.14 (dd, 1H)
6.70 (s, 1H)
4.20 (s, 2H)
2.90 (s, 6H)
2.30 (s, 3H)

Example 13b

5-Chloro-2-[(4-dimethylamino-5-methylpyrid-2-yl)methyl-sulphinyl]benzimidazole (E13b)

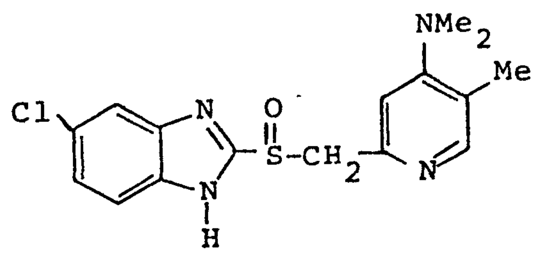

(E.13b)

NMR ($CD_2Cl_2$)

δ 8.05 (s, 1H)
7.64 (d, 1H)
7.59 (d, 1H)
7.27 (dd, 1H)
4.67 (d, 1H)
4.42 (d, 1H)
2.60 (s, 6H)
2.20 (s, 3H)

Example 14a

5-Chloro-2-[(3,5-dimethyl-4-morpholinopyrid-2-yl)-methylthio]benzimidazole (E14a)

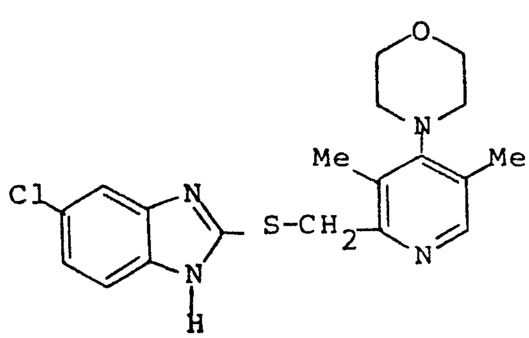

(E.14a)

NMR ($CDCl_3$)

δ 7.98 (s, 1H)
7.40-7.15 (m, 2H)
6.96 (d, 1H)
4.58 (s, 1H)
3.85-3.50 (m, 4H)
3.20-2.90 (m, 4H)
2.34 (s, 3H)
2.27 (s, 3H)

Example 14b

5-Chloro-2-[(3,5-dimethyl-4-morpholinopyrid-2-yl)-methylsulphinyl]benzimidazole (E14b)

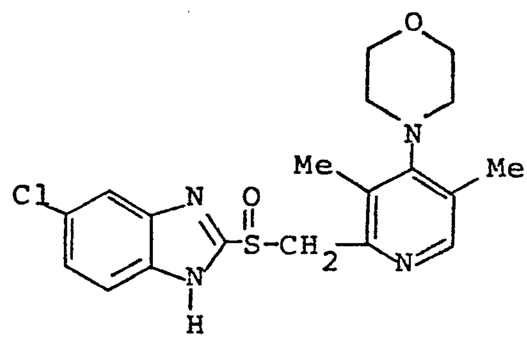

(E.14b)

NMR ($CDCl_3$)

δ 8.16 (s, 1H)
7.65-7.47 (m, 2H)
7.25 (dd, 1H)
4.75 (s, 2H)
3.72 (dd, 4H)
2.95 (dd, 4H)
2.29 (s, 3H)
2.19 (s, 3H)

Example 15a

5-Methyl-2-[(3,5-dimethyl-4-morpholinopyrid-2-yl)-methylthio]benzimidazole (E15a)

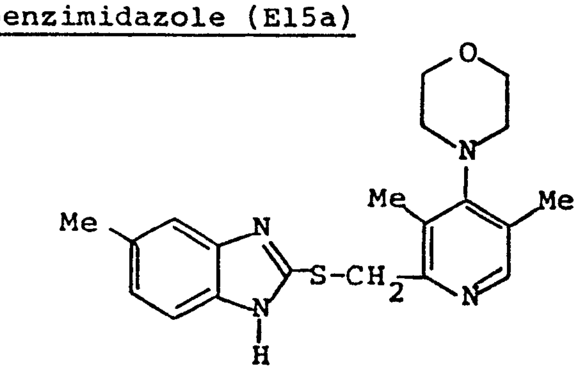

(E.15a)

NMR ($CDCl_3$)

δ 8.06 (s, 1H)
7.40-7.10 (m, 2H)
6.83 (d, 1H)
4.25 (s, 2H)
3.70 (dd, 4H)
3.00 (dd, 4H)
2.38 (s, 3H)
2.29 (s, 3H)
2.23 (s, 3H)

Example 15b

5-Methyl-2-[(3,5-dimethyl-4-morpholino-pyrid-2-yl)-methylsulphinyl]benzimidazole (E15b)

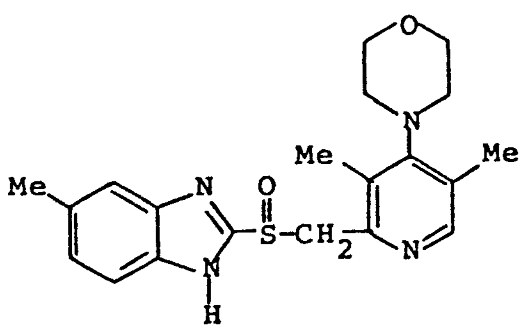

(E.15b)

NMR ($CDCl_3$)

| | | |
|---|---|---|
| δ | 8.16 | (s, 1H) |
| | 7.60-7.25 | (m, 2H) |
| | 7.20 | (dd, 1H) |
| | 4.74 | (s, 2H) |
| | 3.70 | (dd, 4H) |
| | 2.80 | (dd, 4H) |
| | 2.47 | (s, 3H) |
| | 2.27 | (s, 3H) |
| | 2.15 | (s, 3H) |

Example 16a

5-Trifluoromethyl-2-[(3,5-dimethyl-4-morpholino-pyrid-2-yl)methylthio]benzimidazole (E16a)

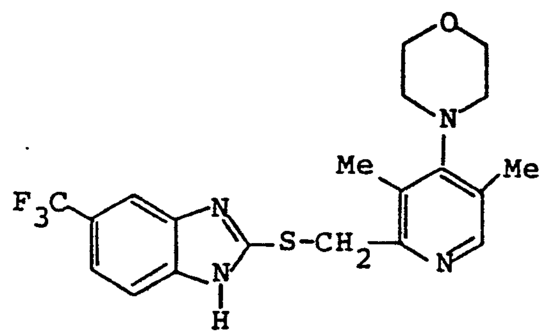

(E.16a)

NMR ($CDCl_3$)

| | | |
|---|---|---|
| δ | 8.05 | (s, 1H) |
| | 7.75-7.10 | (m, 3H) |
| | 4.27 | (s, 2H) |
| | 3.90-3.50 | (m, 4H) |
| | 3.30-2.90 | (m, 4H) |
| | 2.31 | (s, 3H) |
| | 2.26 | (s, 3H) |

Example 16b

5-Trifluoromethyl-2-[(3,5-dimethyl-4-morpholinopyrid-2-yl)methylsulphinyl]benzimidazole (E16b)

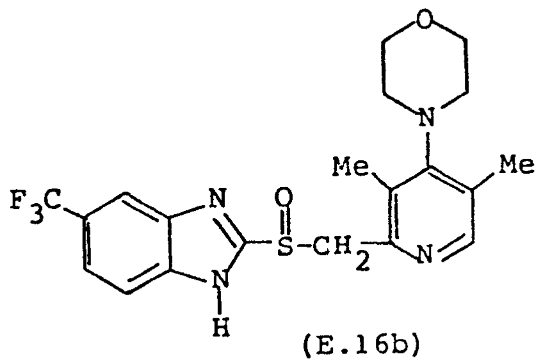

(E.16b)

NMR ($CDCl_3$)

δ 8.15 (s, 1H)
7.90 (brs, 1H)
7.70 (d, 1H)
7.51 (brd, 1H)
4.80 (s, 2H)
3.72 (dd, 4H)
2.92 (dd, 4H)
2.27 (s, 3H)
2.20 (s, 3H)

Example 17a

5-Methoxy-2-[(3,5-dimethyl-4-morpholino-pyrid-2-yl)-methylthio]benzimidazole (E17a)

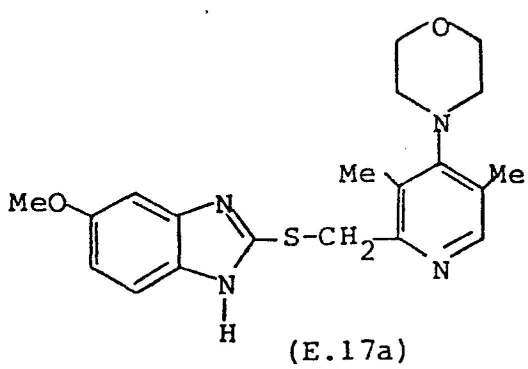

(E.17a)

NMR ($CDCl_3$)

δ 8.16 (s, 1H)
7.33 (d, 1H)
6.97 (d, 1H)
6.72 (dd, 1H)
4.30 (s, 2H)
3.77 (s, 3H)
3.73 (dd, 4H)
3.03 (dd, 4H)
2.31 (s, 3H)
2.27 (s, 3H)

Example 17b

5-Methoxy-2-[(3,5-dimethyl-4-morpholino-pyrid-2yl)-methylsulphinyl]benzimidazole (E17b)

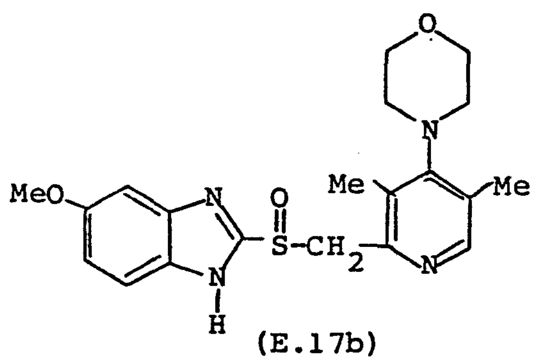

(E.17b)

NMR ($CDCl_3$)

δ 8.16 (s, 1H)
7.70-7.30 (m, 1H)
7.10-6.75 (m, 2H)
4.75 (s, 2H)
3.85 (s, 3H)
3.60 (dd, 4H)
2.91 (dd, 4H)
2.26 (s, 3H)
2.15 (s, 3H)

Example 18a

5,6-Dichloro-2-[(3,5-dimethyl-4-morpholino-pyrid-2-yl)-methylthio]benzimidazole (E18a)

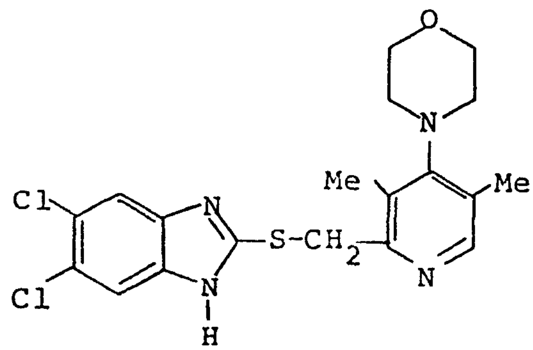

(E.18a)

NMR ($CDCl_3/CD_3OD$)

δ 8.04 (s, 1H)
7.50 (s, 2H)
4.46 (s, 2H)
3.76 (dd, 4H)
3.10 (dd, 4H)
2.37 (s, 3H)
2.30 (s, 3H)

Example 18b

5,6-Dichloro-2-[(3,5-dimethyl-4-morpholino-pyrid-2-yl)-methylsulphinyl]benzimidazole (E18b)

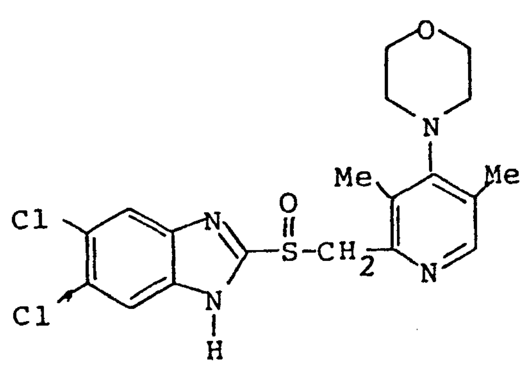

(E.18b)

NMR ($CDCl_3$)

δ 8.16 (s, 1H)
7.69 (s, 2H)
4.75 (s, 2H)
3.75 (dd, 4H)
2.99 (dd, 4H)
2.30 (s, 3H)
2.22 (s, 3H)

Example 19a

4-Cyclopropylcarbonyl-2-[(3,5-dimethyl-4-morpholino-pyrid-2-yl)methylthio]benzimidazole (E19a)

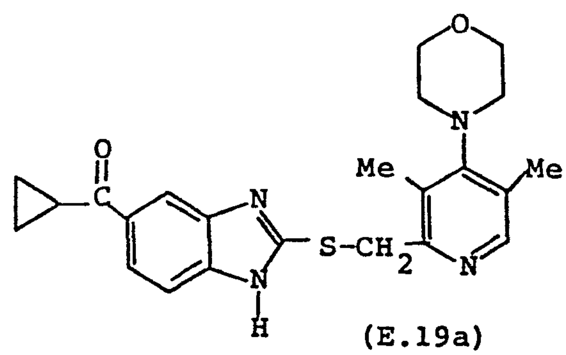

(E.19a)

NMR ($CDCl_3$)

δ 8.10 (brs, 2H)
7.81 (dd, 1H)
7.43 (dd, 1H)
4.33 (s, 2H)
3.73 (dd, 4H)
3.05 (dd, 4H)
2.90-2.40 (m, 1H)
2.32 (s, 3H)
2.28 (s, 3H)
1.40-0.80 (m, 4H)

Example 19b

4-Cyclopropylcarbonyl-2-[(3,5-dimethyl-4-morpholino-pyrid-2-yl)methylthio]benzimidazole (E19b)

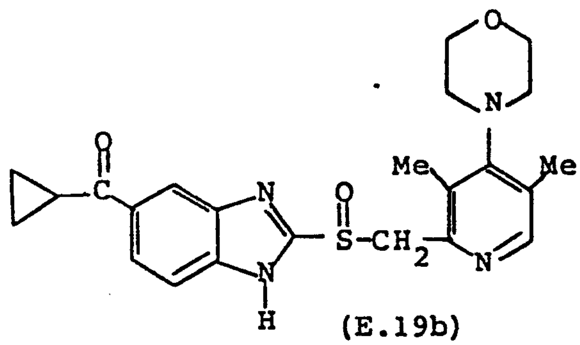

(E.19b)

NMR ($CDCl_3$)

| | | |
|---|---|---|
| δ | 8.38 | (brs, 1H) |
| | 8.17 | (s, 1H) |
| | 8.05 | (dd, 1H) |
| | 7.67 | (d, 1H) |
| | 4.73 | (s, 2H) |
| | 3.75 | (dd, 4H) |
| | 3.00 | (dd, 4H) |
| | 2.85-2.60 | (m, 1H) |
| | 2.30 | (s, 3H) |
| | 2.25 | (s, 3H) |
| | 1.40-0.80 | (m, 4H) |

Example 20a

2-[(5-ethyl-4-morpholino-pyrid-2-yl)methylthio]benzimidazole (E20a)

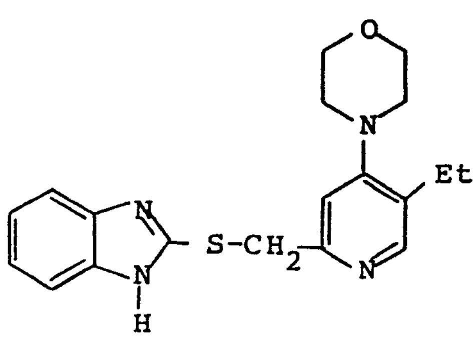

(E.20a)

Example 20b

2-[(5-Ethyl-4-morpholino-pyrid-2-yl)methylsulphinyl]-benzimidazole (E20b)

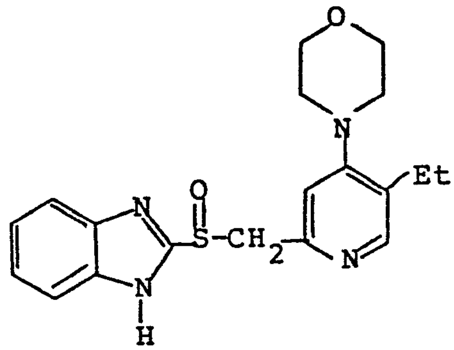

(E.20b)

NMR ($CDCl_3$)

| | | |
|---|---|---|
| δ | 8.20 | (s, 1H) |
| | 7.80-7.50 | (m, 2H) |
| | 7.45-7.15 | (m, 2H) |
| | 6.34 | (s, 1H) |
| | 4.75 | (d, 1H) |
| | 4.51 | (d, 1H) |
| | 3.63 | (dd, 4H) |
| | 2.75-2.25 | (m, 6H) |
| | 1.17 | (t, 3H) |

Example 21a

2-[(3-Methyl-4-morpholino-pyrid-2-yl)methylthio]-benzimidazole (E21a)

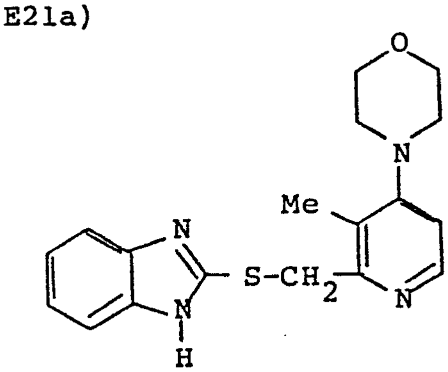

(E.21a)

NMR ($CDCl_3$)

δ 8.25 (d, 1H)
7.60-7.30 (m, 2H)
7.20-6.90 (m, 2H)
6.70 (d, 1H)
4.33 (s, 2H)
3.76 (dd, 4H)
2.90 (dd, 4H)
2.26 (s, 3H)

Example 21b

2-[(3-Methyl-4-morpholino-pyrid-2-yl)methylsulphinyl]-benzimidazole (E21b)

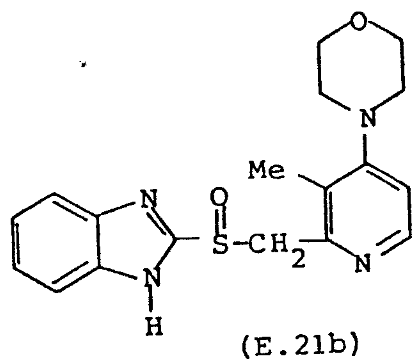

(E.21b)

NMR ($CD_2Cl_2$ + $d^6$DMSO)

δ 8.26 (d, 1H)
7.75-7.45 (m, 2H)
7.35-7.00 (m, 2H)
6.83 (d, 1H)
4.71 (s, 2H)
3.74 (dd, 4H)
2.87 (dd, 4H)
2.21 (s, 3H)

Example 22a

5-Chloro-2-[(3-methyl-4-morpholino-pyrid-2-yl)methylthio]benzimidazole (E22a)

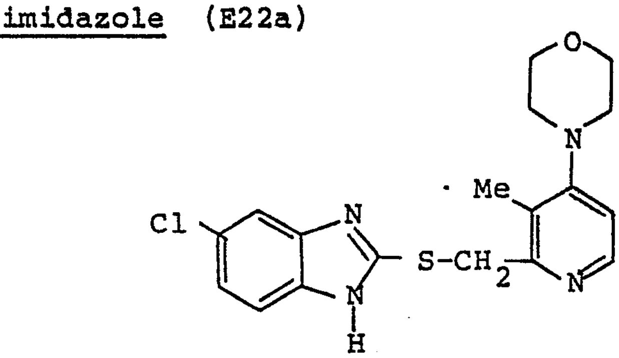

(E.22a)

NMR ($CDCl_3$ + $CD_3OD$)

δ 8.23 (d, 1H)
7.43 (brs, 1H)
7.05 (dd, 1H)
6.76 (d, 1H)
4.43 (s, 2H)
3.83 (dd, 4H)
2.93 (dd, 4H)
2.30 (s, 3H)

Example 22b

5-Chloro-2-[(3-methyl-4-morpholino-pyrid-2-yl)methylsulphinyl]benzimidazole (E22b)

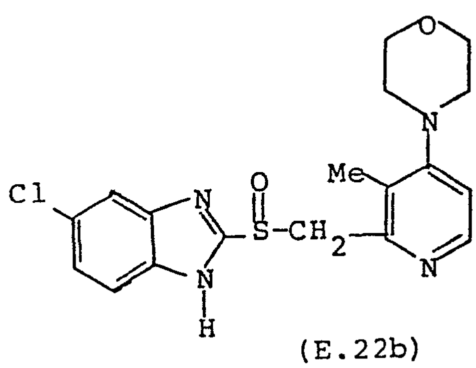

(E.22b)

NMR ($d_6$-DMSO)

| | |
|---|---|
| δ | 8.25 (d, 1H) |
| | 7.75 (s, 1H) |
| | 7.68 (d, 1H) |
| | 7.33 (dd, 1H) |
| | 6.93 (d, 1H) |
| | 4.75 (s, 2H) |
| | 3.75 (dd, 4H) |
| | 2.87 (dd, 4H) |
| | 2.22 (s, 3H) |

Example 23a

2-[(3,5-Dimethyl-4-N-anilino-pyrid-2-yl)methylthio]-benzimidazole (E23a)

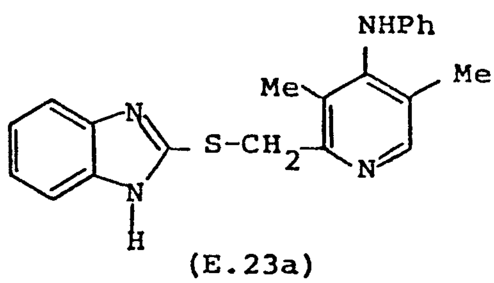

(E.23a)

NMR ($CDCl_3$ + $CD_3OD$)

| | |
|---|---|
| δ | 8.00 (s, 1H) |
| | 7.55-6.40 (m, 9H) |
| | 4.36 (s, 2H) |
| | 2.13 (s, 3H) |
| | 2.06 (s, 3H) |

Example 23b

2-[(3,5-Dimethyl-4-N-anilino-pyrid-2-yl)methyl-sulphinyl]benzimidazole (E23b)

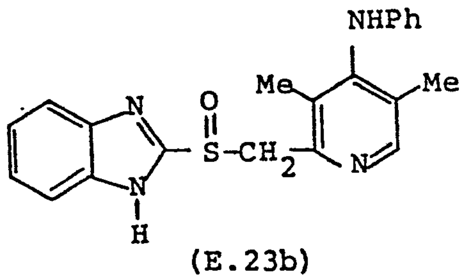

(E.23b)

NMR ($CDCl_3$)

δ 8.23 (s, 1H)
7.80-6.40 (m, 9H)
5.52 (brs, 1H)
4.80 (brs, 2H)
2.08 (s, 6H)

Example 24a

2-[(3,5-Dimethyl-4-N-[N-methylanilino]-pyrid-2-yl-methylthio]benzimidazole (E24a)

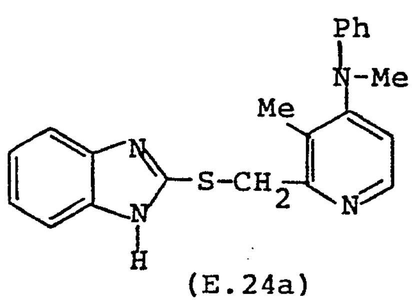

(E.24a)

NMR ($CDCl_3$)

| | | |
|---|---|---|
| δ | 8.30 | (d, 1H) |
| | 7.60-6.60 | (m, 9H) |
| | 4.35 | (s, 2H) |
| | 3.22 | (s, 3H) |
| | 2.03 | (s, 3H) |

Example 24b

2-[(3,5-Dimethyl-4-N-[N-methylanilino]-pyrid-2-yl)-methylsulphinyl]benzimidazole (E24b)

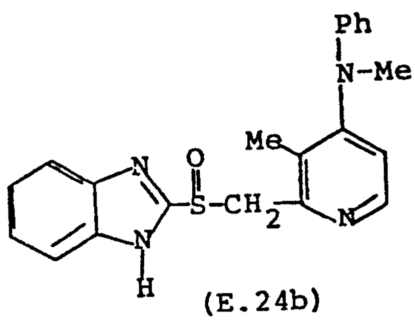

(E.24b)

NMR ($CDCl_3$)

| | | |
|---|---|---|
| δ | 8.42 | (d, 1H) |
| | 7.75-6.60 | (m, 9H) |
| | 4.78 | (s, 2H) |
| | 3.19 | (s, 3H) |
| | 1.95 | (s, 3H) |

Example 25a

2-[(5-Methyl-4-(1-piperidinyl)-pyrimidin-6-yl)methylthio]benzimidazole (E25a)

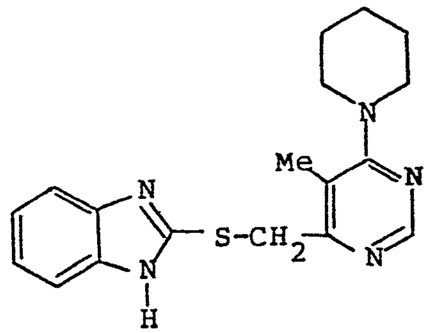

(E.25a)

A solution of D3 (0.56g) and 2-mercaptobenzimidazole (0.32g) in EtOH (30ml) containing 10% aqueous sodium hydroxide (1.7ml) was heated under reflux for 2h. The solvent was then removed in vacuo and the residue partioned between water (5ml) and $CHCl_3$ (100ml). The organic layer was dried ($Na_2SO_4$) and evaporated. Trituration of the residue with $Et_2O$ gave Ela (0.55g) m.p. 145-149°C.

N.M.R. ($CDCl_3$)

δ 8.50 (s, 1H)
7.55-7.25 (m, 2H)
7.25-6.90 (m, 2H)
4.21 (s, 2H)
3.50-3.00 (m, 4H)
2.16 (s, 3H)
1.80-1.40 (m, 6H)

Example 25b

2-[(5-Methyl-4-(1-piperidinyl)-pyrimidin-6-yl)methyl-sulphinyl]benzimidazole (E25b)

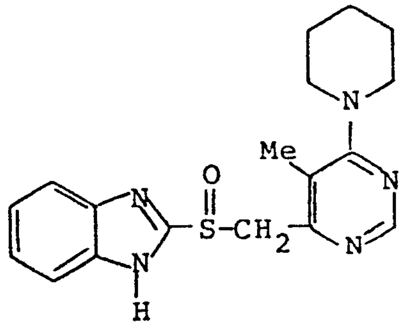

(E.25b)

A solution of E1a (0.55g) in $CHCl_3$ (20ml) at -30°C was treated with m-CPBA (0.34g) for 30min. The $CHCl_3$ solution was washed with aqueous $NaHCO_3$ containing a little $K_2CO_3$, dried ($Na_2SO_4$) and evaporated *in vacuo*. Trituration of the residue with $Et_2O$ gave E1b (0.4g) m.p. 130-132°C (dec)

N.M.R. ($d^6$-DMSO)

| | | |
|---|---|---|
| δ | 8.45 | (s, 1H) |
| | 7.25-7.45 | (m, 4H) |
| | 4.70 | (s, 2H) |
| | 3.20 | (brs, 4H) |
| | 2.15 | (s, 3H) |
| | 1.55 | (brs, 6H) |

Example 26a

2-[(5-Methyl-4-morpholino-pyrimidin-6-yl)methylthio]-benzimidazole (E26a)

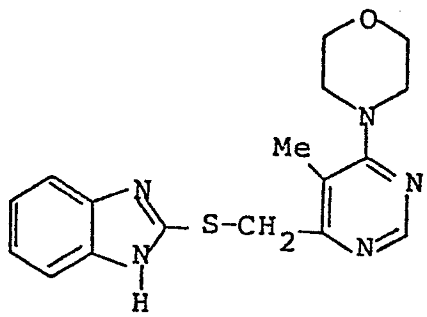

(E.26a)

NMR ($CDCl_3$)

| | | |
|---|---|---|
| δ | 8.56 | (s, 1H) |
| | 7.60-7.30 | (m, 2H) |
| | 7.23-6.97 | (m, 2H) |
| | 4.26 | (s, 2H) |
| | 3.75 | (dd, 4H) |

Example 26b

2-[(5-Methyl-4-morpholino-pyrimidin-6-yl) methylsulphinyl]benzimidazole (E26b)

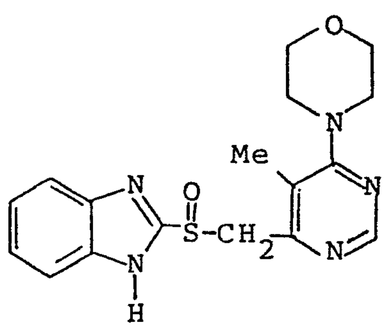

(E.26b)

NMR ($CDCl_3$)

δ 8.60 (s, 1H)
7.90-7.20 (m, 4H)
4.70 (s, 2H)
3.72 (dd, 4H)
3.19 (dd, 4H)
2.06 (s, 3H)

Example 27a

2-[(5-Methyl-4-dimethylamino-pyrimidine-6-yl)methylthio]benzimidazole (E27a)

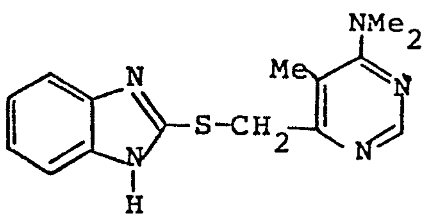

(E.27a)

NMR ($CDCl_3$)

δ 8.49 (s, 1H)
7.65-7.35 (m, 2H)
7.25-6.95 (m, 2H)
4.23 (s, 2H)
2.97 (s, 6H)
2.23 (s, 3H)

Example 27b

2-[(5-Methyl-4-dimethylamino-pyrimidin-6-yl)methyl-sulphinyl]benzimidazole (E27b)

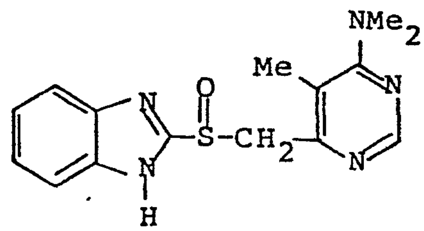

(E.27b)

NMR ($CDCl_3$)

δ 8.55 (s, 1H)
7.85-7.55 (m, 2H)
7.50-7.25 (m, 2H)
4.65 (brs, 2H)
2.97 (s, 6H)
2.17 (s, 3H)

Example 28

The following compound is prepared analogously:

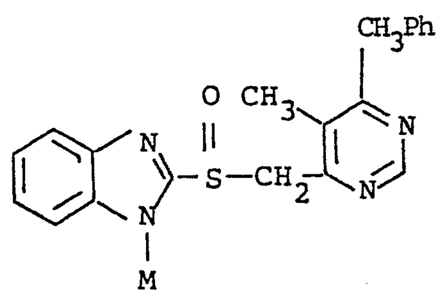

## Pharmacological Data

### The perfused rat stomach preparation

The modified (1) perfused stomach preparation (2) ofthe urethane (25% solution) anaesthetized rat, maintained at 37°C, allows the continuous measurement of pH during basal and stimulated acid secretion.

The lumen of the stomach of male Wistar rats (approximately 200 g bodyweight) was perfused, via a cannula designed to reduce the dead space of the stomach, with 5% glucose solution (37°C) at the rate of 3 ml/min. The perusate was forced over the surface of the secretory mucose only, the antrum being excluded. The effluent then passed over a microflow type glass pH electrode via a collecting funnel situated in the non-glandular forestomach.

The secretagogue carbachol or histamine was administered as a constant intravenous infusion to produce a stady rate of acid secretion. Test compounds were administered in solution as bolus intravenous injections and any effect on th pH of the perfusate noted. The perfusate Ph was recorded on a potentiometric recorder and anti-secretory responses were measure in terms of the maximal reduction in hydrogen-ion concentration expressed as a percentage of the 'control' concentrations.

Results

| Example No. | Dose µmol/kg | Inhibition % |
|---|---|---|
| 2 | 1 | 37 |
| 6b | 2 | 50 |
| 8b | 0.5 | 72 |
| 11b | 2 | 37 |
| 14b | 1 | 60 |
| 21b | 2 | 87 |
| 23b | 5 | 86 |
| 24b | 2 | 100 |
| 25b | 2 | 84 |
| 26b | 2.5 | 84 |
| 27b | 2.5 | 92 |

References

1. Parsons, M.E. (1970)
ph.D. Thesis, University of London

2. Ghosh, M.N. and Schild,H.O. (1958)
Br. J. Pharmacol., 13, 54-61

CLAIMS: C

1. A compound of the formula I a salt, quaternized derivate or a solvate thereof:

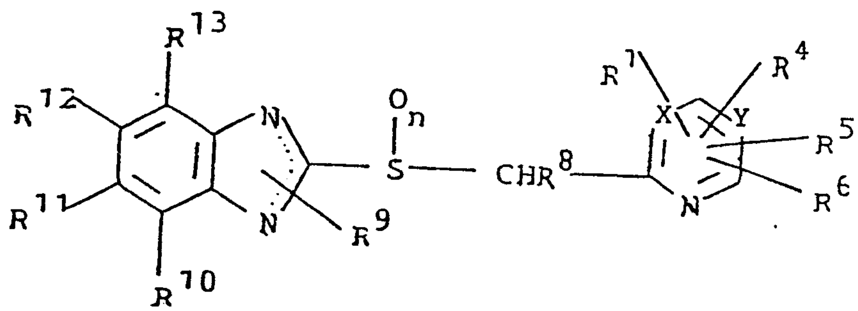

I

wherein:

n represents 0 or 1;

X and Y are both C- or one of X and Y is N and the other is CH;

$R^1$ either represents $NR^2R^3$, in which $R^2$ and $R^3$ independently represent a hydrogen atom or an alkyl, aryl, aralkyl, or acyl group, or N, $R^2$ and $R^3$ together form a saturated or unsaturated, substituted or unsubstituted ring, optionally containing one or more further hetero atoms; and

$R^4$, $R^5$, (and $R^6$ present when X and Y are both C-) each represent hydrogen or a substituent, or

$R_1$ represents $NR^2R^7$ in which $R^2$ represents a hydrogen atom or an alkyl, aryl, aralkyl, or acyl group, and $R^7$ and one of $R^4$, $R^5$, (and $R^6$ when present) on a carbon atom of the pyridine ring adjacent to that carrying $R^1$ are linked to complete a saturated or unsaturated, substituted or unsubstituted ring, optionally containing one or more further hetero atoms, and the

others of $R^4$, $R^5$ (and $R^6$ when present) each represent hydrogen or a substituent;

$R^8$ represents a hydrogen atom, an alkyl group, or, when X is $C^-$ and one of $R^4$, $R^5$ or $R^6$ is a substituent, $R^8$ completes a ring therewith;

$R^9$ represents a hydrogen atom, or an alkanoyl, alkoxycarbonyl, aryloxycarbonyl, aralkyloxycarbonyl, aralkanoyl, aroyl, alkylsulphonyl, arylsulphonyl, aralkylsulphonyl, or aralkyl group; and

$R^{10}$, $R^{11}$, $R^{12}$ and $R^{13}$ each independently represents hydrogen or a substituent.

2. A compound according to claim 1 wherein $R^4$, $R^5$ and $R^6$ independently represent a halogen atom, or a $C_{1-6}$ alkyl, $C_{1-6}$ alkoxy, $C_{1-6}$ alkylthio, $C_{1-6}$ alkoxy$C_{1-6}$ alkoxy, $C_{2-7}$ acyloxy, cyano, nitro, carboxy, esterified carboxy, or $C_{2-7}$ acyl group, or $NR^2R^3$ wherein $R^2$ and $R^3$ are as defined in claim 1; or one of $R^4$, $R^5$ and $R^6$ is linked to an $R^7$ group of $R^1$ to form a ring as specified in claim 1 and one or both of the others represent a substituent, one or each independently represents a halogen atom, or a $C_{1-6}$ alkyl, $C_{1-6}$ alkoxy, $C_{1-6}$ alkylthio, $C_{1-6}$ alkoxy$C_{1-6}$ alkoxy, or $C_{2-7}$ acyloxy group, or $NR^2R^3$ as defined in claim 1; or one of $R^4$, $R^5$, and $R^6$ is linked to an $R_7$ group of $R^1$ to form a ring as defined in claim 1, and the remaining two of $R^4$, $R^5$ and $R^6$ are adjacent to each other, one of the remaining two may represent $NR^2R^7$, where $R^2$ and $R^7$ are as defined in claim 1, $R^7$ being linked to the other of the remaining two, to form a further ring as defined in claim 1.

3. A compound according to claim 1 or 2 wherein $R^1$ is in the 4-position on the pyr(im)idine ring.

4. A compound according to any one of claims 1, 2 or 3 wherein $R^2$ and $R^3$ are both methyl or ethyl groups, $R^2$ is hydrogen and $R^3$ is benzyl, $R^2$ is methyl and $R^3$ is phenyl, or $NR^2R^3$ together form pyrrolidino or morpholino.

5. A compound according to any one of claims 1 to 4 wherein $R^4$, $R^5$ and $R^6$ are hydrogen or methyl in the 3- or 5- positions in the pyr(im)idine ring.

6. A compound according to claim 1 of formula II:

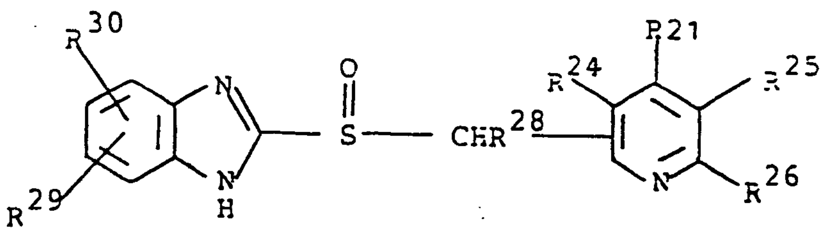

(II)

wherein $R^{21}$ represents dimethylamino, piperidino, pyrrolidino or morpholino; $R^{24}$, $R^{25}$ and $R^{26}$ each independently represents hydrogen or alkyl, especially methyl; $R^{28}$ represents hydrogen or methyl, and $R^{29}$ and $R^{30}$ each independently represents hydrogen, chlorine, bromine, fluorine, or an amino, methyl, trifluoromethyl, methoxy, ethoxycarbonyl or acetyl group.

7. A compound according to claim 1 of formula III:

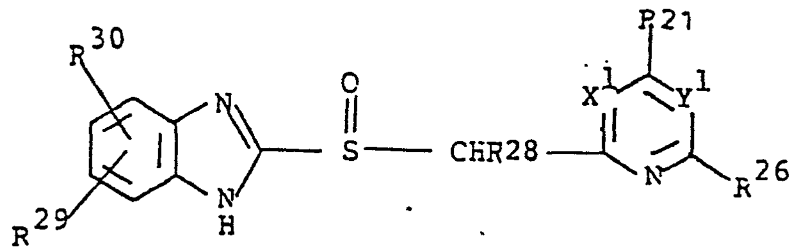

(III)

wherein $X^1$ is N and $Y^1$ is $CR^{25}$ where $R^{25}$ is as defined in claim 6 or $X^1$ is $CR^{24}$ where $R^{24}$ is as defined in claim 6 and $Y^1$ is CN

8. 2-[(3,5-dimethyl-4-morpholino-pyrid-2-yl)methylsulphinyl]benzimidazole.

9. A process for the preparation of a compound according to any one of claims 1 to 8 which process comprises reaction of a 2-substituted benzimidazole with a substituted pyridine or pyrimidine wherein the substituents on the two compounds react to provide a linkage of the formula IV

$$-S(O)_nCHR^8- \quad (IV)$$

with the sulphur atom on the 2-position of the benzimidazole nucleus, where n and $R^8$ have the meanings given above, and, if desired or required, providing or removing other substituents in or from the resulting compound and, if desired or required, removing protective groups from other substituents on the resulting compound and, if desired or required, oxidizing or reducing the resulting compound, and, if desired or required, converting a free base into an

acid addition salt, or quaternizing or solvating the resulting compound.

10. A pharmaceutical composition comprising a compound according to any one of claims 1 to 9 and a pharmaceutically acceptable carrier.

11. A compound according to any one of claims 1 to 9 for use in the treatment of disorders caused or exacerabated by excess gastric acid secretion.

European Patent Office

0178438

Application number

# EUROPEAN SEARCH REPORT

EP 85 11 1067

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| E | EP-A-0 150 586 (UPJOHN)<br>* Pages 3,4,6-9,26-28,31; claim 7 *<br>----- | 1-6,8-11 | C 07 D 401/12<br>C 07 D 403/12<br>C 07 D 471/04<br>A 61 K 31/415 |
| | | | TECHNICAL FIELDS SEARCHED (Int. Cl 4) |
| | | | C 07 D 401/00<br>C 07 D 403/00<br>C 07 D 471/00<br>A 61 K 31/00 |

The present search report has been drawn up for all claims

| Place of search | Date of completion of the search | Examiner |
|---|---|---|
| THE HAGUE | 10-01-1986 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82